# EUROPÄISCHE PATENTSCHRIFT

(11) **EP 2 004 115 B1**
(45) Veröffentlichungstag und Bekanntmachung des Hinweises auf die Patenterteilung: **18.03.2015**
(21) Anmeldenummer: 07722239.6
(22) Anmeldetag: 05.04.2007
(51) Int. Cl.: A61F 9/04, A61F 9/00

(54) **HALTEVORRICHTUNG FÜR EINE MEMBRAN UND TRÄGER FÜR DIE HALTEVORRICHTUNG AN EINEM AUGE**
HOLDING DEVICE FOR A MEMBRANE AND SUPPORT FOR THE HOLDING DEVICE TO AN EYE
DISPOSITIF DE RETENUE DE MEMBRANE ET SUPPORT POUR CE DISPOSITIF DE RETENUE SUR UN OEIL

(30) Priorität: 13.04.2006 DE 102006019017
(43) Veröffentlichungstag der Anmeldung: 24.12.2008
(73) Patentinhaber: Leibniz-Institut für Polymerforschung Dresden e.V., 01069 Dresden (DE); Klinikum Chemnitz gGmbH, 09116 Chemnitz (DE)
(72) Erfinder: ENGELMANN, Katrin, 01326 Dresden (DE); KÖRBER, Heinz, 01324 Dresden (DE); WERNER, Carsten, 01219 Dresden (DE)
(74) Vertreter: Sperling, Thomas
(86) Internationale Anmeldenummer: PCT/DE2007/000681
(87) Internationale Veröffentlichungsnummer: WO 2007/118470

(56) Entgegenhaltungen:
- WO-A-03/077794
- US-A- 3 392 725
- US-A- 6 123 081

## Beschreibung

Die Erfindung betrifft eine Haltevorrichtung für eine Membran in einer medizinischen Anwendung am Auge.

Membranen werden beispielsweise bei Behandlungen von Augenoberflächen auf diese aufgebracht und dabei am bzw. auf dem Auge fixiert.

Es ist bekannt, Membranen zur Wundheilung auf der Augenoberfläche durch Verkleben oder durch Vernähen anzuordnen. Diese Art der Befestigung kann zu Problemen führen, da die Befestigung der Membran in der Regel in von der erforderlichen Wundheilung nicht betroffenen, gesunden Bereichen der Augenoberfläche erfolgt und in diesen beschwerdefreien Bereichen in Einzelfällen zu Reizungen, Blutungen, Vernarbungen oder Ähnlichem führen kann. Des Weiteren kann es durch den Lidschlag zu Kräften auf die auf der Augenoberfläche platzierte Membran kommen, die zu einer Verschiebung oder gar zu einem Abreißen der Membran mit der Notwendigkeit einer neuerlichen Befestigung führen können. Weiter bekannt ist es, eine vernetzte Membran aus einem Amnionhäutchen einzusetzen.

Die WO 2003/077 794 A2 zeigt verschiedene Vorrichtungen zur Halterung von Membranen an geeigneten Halteeinrichtungen, unter anderem eine Einrichtung, bei der eine Membran zwischen zwei in einer Schnappverbindung verbundenen Ringen sandwichartig befestigt ist. Die Membran wird dabei zwischen Klemmflächen der beiden Ringe fixiert, wobei eine der Klemmflächen gezackt ausgebildet ist. Die stirnseitigen, die Klemmflächen bildenden Randabschnitte der beiden Ringe werden so umgebogen oder konisch ausgebildet, dass die Membran zwischen diesen Randabschnitten fixiert ist.

Ausgehend von diesem Stand der Technik liegt der Erfindung die Aufgabe zugrunde, eine Membran in einfacher und sicherer Weise auf einer Augenoberfläche anzuordnen, wobei sowohl die vorbereitende Behandlung der Membran wie auch das Haltern in dem Auge mit einer möglichst geringen Beanspruchung erfolgen soll.

Zur Lösung dieser Aufgabe wird eine Haltevorrichtung mit den Merkmalen des Patentanspruchs 1 vorgeschlagen.

Die erfindungsgemäße Haltevorrichtung, mit der Anordnung der Membran an zwei konzentrisch aneinander angeordneten Ringen, wobei ein erster Ring als Innenring von einer Aufnahme an der Innenseite eines zweiten Rings als Außenring aufgenommen ist, bietet die Möglichkeit, die Membran in der Aufnahme ohne die Notwendigkeit weiterer Befestigungsmittel zwischen den beiden Ringen verliersicher einzuklemmen. Die Membran ist dabei an ihrem Umfang zwischen den beiden Ringen aufgespannt und überspannt frei die gemeinsame Öffnung der konzentrisch angeordneten Ringe. Bei einem Aufbringen auf das menschliche Auge kann der frei gespannte Abschnitt der Membran unter Spannung in Kontakt mit der Augenoberfläche treten. Zur Anpassung an die Wölbung des menschlichen Auges ist die gemeinsame Innenseite, die bei der Anordnung des Innenrings in der Aufnahme des Außenrings entsteht, als Kegelstumpf ausgebildet, der sich in Richtung der Membran verjüngt. Der Innenring fluchtet mit seiner Innenseite mit der Innenseite des Außenrings, in dem er aufgenommen ist, so dass eine gemeinsame kegelstumpfförmige Innenfläche der Haltevorrichtung gebildet ist. Auf diese Weise ist eine nahtlose und damit gewebeschonende und passgenaue Aufbringung von Membranen auf die Augenoberfläche möglich.

Mit Vorteil ist dabei der Innenring weitgehend formstabil, während der Außenring elastisch ist. Die Stabilität der aus den beiden aneinander angeordneten Ringen bestehenden Haltevorrichtung beruht dabei auf dem Innenring, während der mit den empfindlichen Teilen des Auges bei der medizinischen Anwendung in Kontakt tretende Außenring elastisch und damit weicher ausgebildet ist, um Reizung oder gar Verletzungen an dem schon geschädigten Auge zu vermeiden. Die elastische Ausbildung des Außenrings ermöglicht es auch, den Innenring in einfacher Weise in der Aufnahme des Außenrings zu platzieren, indem der elastische Außenring über den formstabilen Innenring gestülpt wird. Da auch die Membran bei diesem Anordnen des Innenrings zwischen den beiden Ringen kraftschlüssig gehaltert wird, wird auch das Risiko der Beschädigung der Membran durch die elastisch-weiche Ausbildung des einen Rings minimiert. Dabei ist es weiterhin sinnvoll, den formstabilen Innenring zum Schutz des Auges an dessen Außen- und Oberseite weitgehend vollständig durch den Außenring zu überdecken. Die Aufnahme in dem Außenring hat in dieser Ausführung nur an der Innenseite des Rings eine Öffnung, die bündig mit der Innenseite des Außenrings von der Innenseite des Innenrings geschlossen ist, so dass keine Kanten oder Überstände auftreten.

Hierzu weist der Außenring erfindungsgemäß eine weitgehend als Dreieck ausgebildete Querschnittsfläche mit einer keilförmigen Ausnehmung für den Innenring auf. Dabei ist eine der Seiten des Dreiecks, beginnend im Bereich einer der Spitzen des Dreiecks, teilweise von einer Seite der keilförmigen Ausnehmung gebildet. Das heißt, dass in der montierten Position der beiden Ringe ein Teil dieser Seite von der Innenseite des Innenrings gebildet ist. Die zu dem Gegenwinkel dieser Seite des Dreiecks gehörende Spitze ist als abgerundete Kante ausgebildet, da diese Kante den Teil der Haltevorrichtung bildet, die beim Einsetzen in das Auge den größten Abstand zur Augenoberfläche aufweist und von der die größte Belastung für das Auge ausgeht. Daher ist es in diesem Bereich vorteilhaft, Spitzen und Kanten zu vermeiden.

Weiterhin hat es sich als günstig herausgestellt, den Außenring an seinem unteren Abschnitt dem Innenring überstehend und spitz auslaufend auszubilden. Die Haltevorrichtung ragt in diesem Bereich beim Einsetzen in das Auge besonders tief in die Augenhöhle hinein, wobei der Abstand zwischen der Bindehaut und dem Augapfel geringer wird. Um Reizungen zu vermeiden, verringert sich durch das spitze Auslaufen die Dicke des Außenrings bei einer maximalen Anlagefläche. Der entstehende scharfkantige Abschluss bietet keine Angriffsfläche bei Bewegungen der Haltevorrichtung an bzw. auf dem Auge.

Gleichzeitig weist der Innenring weiter mit Vorteil eine keilförmige Querschnittsfläche auf, deren Spitze in Richtung der zwischen den beiden Ringen eingespannten Membran zeigt, die der Oberseite des Innenrings aufliegt. In dem Bereich, in dem die Membran zwischen die beiden Ringen eingeführt wird, sind diese jeweils spitz aufeinander zulaufend und einen Spalt bildend ausgebildet. Damit fällt der Kraftaufwand beim Überziehen des elastischen Außenrings über den formstabilen Innenring ebenso gering aus wie die Richtungsänderung, die zum Einführen der Membran zwischen die beiden Ringe notwendig ist.

Der Öffnungswinkel der gemeinsamen kegelstumpfförmigen Innenfläche der beiden zusammenwirkenden Ringe liegt in sinnvoller Weise im Bereich der Wölbung eines menschlichen Augapfels und beträgt bevorzugt 97°, da dann die gesamte Halterungsvorrichtung dem Auge möglichst passgenau anliegt und die Membran mit ihrer freien Fläche im Bereich der gemeinsamen Ringöffnung der Augenoberfläche zur Anlage gebracht werden kann. Durch die möglichst passgenaue Anlage der Innenseite der beiden Ringe tritt der Augapfel teilweise durch die gemeinsame konzentrische Ringöffnung hindurch und ist damit in Kontakt mit der Membran, um insbesondere für die zentral befindliche Hornhaut ihre Heilwirkung entfalten zu können.

Besondere Eignung für diese medizinische Anwendung am Auge weist eine Amnionmembran auf, die aus der der menschlichen Plazenta gewonnen wird.

Vorteilhafte Ausgestaltungen der Erfindung bestehen darin, die eingesetzten Membranen mit Wachstumsfaktoren zu beladen.

Zur Ausbildung der Ringe haben sich ein thermoplastischer, formstabiler Kunststoff für den Innenring und ein elastisches Silikon für den Außenring als besonders vorteilhaft herausgestellt. Beide Materialien ermöglichen eine einfache Herstellung der Haltevorrichtung und ihre Anwendung ist gesundheitlich unbedenklich.

Die der Erfindung zugrunde liegende Aufgabe wird des Weiteren durch einen Träger mit den Merkmalen des Patentanspruchs 9 gelöst. Der erfindungsgemäße Träger für die vorgenannte Haltevorrichtung weist einen Tragkörper auf. Der Tragkörper ist ein Zylinder mit kreisförmiger Querschnittsfläche und einer nach außen gewölbten Stirnseite. Die nach außen gerichtete Wölbung der Stirnseite entspricht dabei in etwa der Kontur und Wölbung eines menschlichen Augapfels im Bereich der Hornhaut.

Der Durchmesser der Stirnseite an ihrer Basis ist mit dem Außendurchmesser des Innenrings der Haltevorrichtung weitgehend identisch.

Mit dieser Ausgestaltung des Trägers kann die Haltevorrichtung mit der Membran an dem Träger so angeordnet werden, dass die Membran dem Tragkörper zur Anlage gebracht werden kann, wobei der Tragkörper weitgehend die Kontur einer Augenoberfläche aufweist. Die Membran wird auf diese Weise vorpositioniert und kann in der Anwendungsposition befindlich präpariert und/oder konserviert werden. Damit sind Formveränderungen, die zu einer verminderten Anpassung an die Augenoberfläche führen können, deutlich reduziert. Auf dem Träger befindlich kann das gebrauchsfertige Konstrukt der die Membran spannenden Haltevorrichtung bis zur Operation gebrauchsfertig aufbewahrt und dann während der Operation in einfacher Weise zum Einsatz gebracht werden. Der Tragkörper selbst kann an einer Grundplatte gehaltert werden.

Zur weiteren Adaption des Trägers an die Form der Haltevorrichtung sind die Randbereiche der Stirnseite in sinnvoller Weiterbildung kegelstumpfförmig ausgebildet, wobei es sinnvoll ist, wenn der Öffnungswinkel des Kegelstumpfes dem Öffnungswinkel des Kegelstumpfes der Haltevorrichtung entspricht.

In einer alternativen Ausführungsform weist der Tragkörper des Trägers eine umlaufende Nut im Bereich der Basis der Stirnseite auf, wobei die Nut mindestens die Tiefe der radialen Erstreckung des Innenrings der Haltevorrichtung hat. Mit dieser Ausbildung liegt die Haltevorrichtung dem Tragkörper weitgehend nur mit der gespannten Membran auf. Diese unterliegt allein durch die auf die beiden Ringe wirkenden Schwerkraft einer permanenten Zugspannung, die Verwerfungen, Falten oder Ähnliches bei der Membran verhindert und damit bei Anwendung der Haltevorrichtung im menschlichen Auge eine weitgehend glatte Anlage an die Augenoberfläche ermöglicht.

Mit der vorliegenden Erfindung ist es möglich, die Membran mittels der erfinderischen Haltevorrichtung am zu behandelnden Auge zu platzieren, wobei die Haltevorrichtung in dem Auge allein von dem Lid des Auges auf der Hornhaut und der Bindehaut gehalten und die Membran dabei an der Augenoberfläche zur glatten Auflage gebracht wird. Eine zusätzliche Belastung des Auges durch eine Verbindung der Membran mit dem Auge ist nicht notwendig. Auch findet keine weitere Belastung der Membran durch ein solches Verbinden statt, die zu unerwünschten Veränderungen oder Beschädigungen an der Membran führen könnten. Die Membran wird im Wege der Augenoperation nicht mehr verändert, sondern mit der Halterung, in der sie dem Operateur präsentiert wird, in das Auge eingesetzt. Ein Vernähen, Verkleben oder ein ähnlich inversives Verbinden mit Teilen des Auges ist nicht erforderlich.

Die Haltevorrichtung mit der aufgespannten Membran steht bereits vor der Operation gebrauchsfertig zur Verfügung. Die eigentliche Operation am Auge kann damit auf das Platzieren der Haltevorrichtung mit der Membran und die hierzu notwendigen Maßnahmen beschränkt werden. Veränderungen an der Haltevorrichtung oder an der Membran sind dabei nicht mehr erforderlich, sondern die Haltevorrichtung mit der Membran wird als fertige Einheit in das Auge eingesetzt.

Es ist weiterhin möglich, die Präparation der Membran vor dem Einsetzen in das Auge in der Haltevorrichtung vorzunehmen. Die fertig präparierte Membran unterliegt dann keinen weiteren Belastungen durch Einsetzen oder Fixierung in einer Halterung.

Aus dem gleichen Grund ist es zu empfehlen, die Membran vor dem Einsetzen in das Auge in der Haltevorrichtung zu konservieren. Bei einer Konservierung, beispielsweise durch Tiefgefrieren der Membran in einem Nährmedium, können durch das vorgeschaltete Aufspannen Fältelungen der Membran während der Konservierung vermieden werden, die sich bei dem Aufbringen der Membran auf das Auge kaum wieder vollständig entfernen lassen.

Eine für diese medizinische Anwendung mit der erfinderischen Haltevorrichtung besonders geeignete Membran ist eine Amnionmembran.

Weitere Vorteile und Merkmale der Erfindung können der nachstehenden Beschreibung zu den Ausführungsbeispielen sowie den einzelnen Patentansprüchen entnommen werden.

In der Zeichnung zeigt:
- Fig. 1: eine Haltevorrichtung für eine Membran in perspektivischer Ansicht,
- Fig. 2: die Haltevorrichtung gemäß Figur 1 mit einer eingespannten Membran in geschnittener Darstellung,
- Fig. 3: einen Träger für die Haltevorrichtung in perspektivischer Ansicht,
- Fig. 4: den Träger gemäß Figur 3 in quergeschnittener Darstellung gemäß Linie IV-IV,
- Fig. 5: den Detailausschnitt "C" gemäß Figur 4,
- Fig. 6: einen Träger für die Haltevorrichtung in einer alternativen Ausführung in quergeschnittener Darstellung.

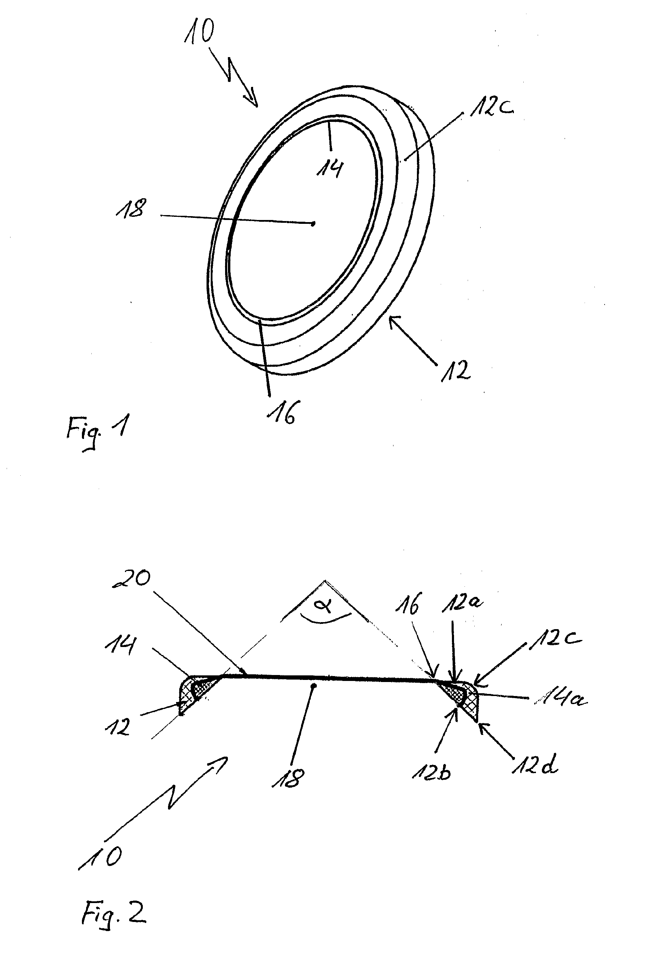

Figur 1 zeigt eine Haltevorrichtung 10, in die eine Membran aufgenommen und mit der Haltevorrichtung in einem menschlichen Auge für eine medizinische Behandlung in Kontakt mit der Augenoberfläche gebracht werden kann.

Durch das Aufbringen einer Amnionmembran, gewonnen aus der Plazenta nach einer Kaiserschnittgeburt, können Wundheilungsstörungen, insbesondere des kornealen Epithels, nach Verletzung oder sonstiger Schädigung der Hornhaut zumindest gelindert werden.

Um eine in Figur 1 nicht gezeigte Membran hierzu an der Haltevorrichtung anzuordnen, das heißt aufspannen zu können, besteht die Haltevorrichtung aus einem Außenring 12, der so elastisch ist, dass er über einen formstabilen Innenring 14, von dem in der Figur 1 nur eine Spitze zu sehen ist, gezogen werden kann. Im Bereich der Spitze des Innenrings 14 bildet dieser in der montierten Position mit dem Außenring 12 einen flexiblen Spalt 16 aus, in dem eine Membran aufgenommen und fixiert werden kann. Aufgrund der elastischen Ausbildung des Außenrings 12 ist der Spalt 16 flexibel und die Membran unter Spannung halterbar.

Der Innenring 14 und der Außenring 12 sind konzentrisch aneinander angeordnet und weisen eine gemeinsame, konzentrische Öffnung 18 auf, die dann von einer Membran überspannt werden kann. Beide Ringe sind dabei als rotationssymmetrische Körper ausgebildet.

Figur 2 zeigt die Haltevorrichtung 10 so, wie sie in das nicht gezeigte Auge eingebracht wird. Die Haltevorrichtung weist als Membran eine Amnionmembran 20 auf, die auf den formstabilen Innenring 14 aus PEEK aufgelegt und durch den elastischen, aus Silikon gefertigten Außenring 12 fixiert ist. Der formstabile Innenring 14 ist mit einem keilförmigen Querschnitt ausgebildet, wobei die Spitze des Keils in den Bereich des Spaltes 16 an der Oberseite der Haltevorrichtung weist. Der elastische Außenring 12 ist mit einer Aufnahme 12a ausgebildet und kann aufgrund seiner Elastizität so über den Innenring 14 gezogen werden, dass der Innenring 14 bündig mit der Innenfläche 12b des Außenrings 12 abschließend in dieser Aufnahme 12a aufgenommen ist. Mit Ausnahme des von dem Innenring 14 gebildeten Teils der Innenfläche 12b und dem Spalt 16 zwischen den beiden Ringen umschließt der Außenring 12 den Innenring 14 vollständig.

Aufgrund seiner elastischen Ausbildung umschließt der Außenring den Innenring unter Spannung, so dass der formstabile Innenring nur mit entsprechendem Kraftaufwand aus seiner Verbindung mit dem Außenring 12 gelöst werden kann, wobei die Aufnahme 12a in Bezug auf ein Herausnehmen des Innenrings aus der Aufnahme hinterschnitten ausgebildet ist und die in die Aufnahme 12a ragende Kante 14a des Innenrings 14 abgerundet ist, um das Risiko der Beschädigung des Außenrings 12 im Bereich seiner Aufnahme 12a durch den formstabilen Innenring zu reduzieren.

Die kreisförmige Amnionmembran 20 ist damit ohne weitere mechanische Befestigungsmittel an ihrem Umfang zwischen der Oberseite des Innenrings 14 und dem diesen umschließenden Außenring 12 eingeklemmt und erstreckt sich über die gesamte gemeinsame Öffnung 18 der beiden Ringe, wobei die Amnionmembran unter einer gewissen Vorspannung an den Ringen befestigt ist. Im Bereich der konzentrischen, gemeinsamen Öffnung 18 der Ringe 12, 14 ist die Amnionmembran 20 frei gespannt und kann zu medizinischen Anwendungen zur Anlage an die Oberfläche eines menschlichen Auges gebracht werden.

Der Außenring 12 weist ebenfalls eine weitgehend dreieckförmige Querschnittsfläche auf, wobei die Spitze dieses an sich rechtwinkligen Dreiecks zu dessen Gegenwinkel auf die die Hypotenuse des Dreiecks bildende Innenseite 12b abgerundet ist, da die durch diese Spitze gebildete, umlaufende Kante 12c des Außenrings 12 bei der Positionierung der Haltevorrichtung 10 in dem Auge direkt unter dem Augenlid platziert und von diesem gehalten wird. Mit dieser Querschnittsfläche bildet die Innenfläche 12b des rotationssymmetrischen Außenrings 12 einen an der Ober- und Unterseite offenen Kegelstumpf, der sich nach oben verjüngt und einen an die Wölbung eines menschlichen Auges angepassten Öffnungswinkel α von ca. 97° aufweist.

Der Außenring 12 weist an der offenen Unterseite des Kegelstumpfs eine spitz zulaufende Kante 12d auf, mit der die Haltevorrichtung 10 auf dem Auge abgestützt wird. Die kreisförmig umlaufende Kante 12d des elastischen, aus Silikon bestehenden Außenrings 12 hat einen Durchmesser von 19,8 mm gegenüber einem Durchmesser von 17,4 mm im Bereich der äußeren Kante der Aufnahme 12a an der Innenfläche 12b, so dass die Haltevorrichtung 10 in dem Randbereich der Hornhaut des Auges nur mit dem weicheren Außenring zur Auflage und die Oberfläche des nach außen gewölbten Auges innerhalb der umlaufenden Kante 12d der Haltevorrichtung 10 mit der Unterseite der Amnionmembran 20 zur Anlage gebracht werden kann. Die Öffnung 18 hat im Bereich des oberen Abschlusses der Haltevorrichtung 10, in dem die Amnionmembran gespannt ist, einen Durchmesser von 14,2 mm.

In der Position der Haltevorrichtung in dem Auge tritt diese in Kontakt mit der Augenoberfläche, wobei die Kante 12d unter das Lid geschoben ist. Das die Augenoberfläche überdeckende, bewegbare Lid hält die Haltevorrichtung 10 durch dauerhafte Überdeckung des Bereiches der abgerundeten Kante 12c der Haltevorrichtung, wobei bei einem Lidschlag die gesamte Haltevorrichtung mit der Oberseite der Amnionmembran 20 von dem Lid überdeckt ist. Die maximale Dicke der Haltevorrichtung 10 gegenüber der Augenoberfläche beträgt 1,5 mm, die sich die abgerundete Kante 12c über die Innenfläche 12b des Außenrings 12 erhebt.

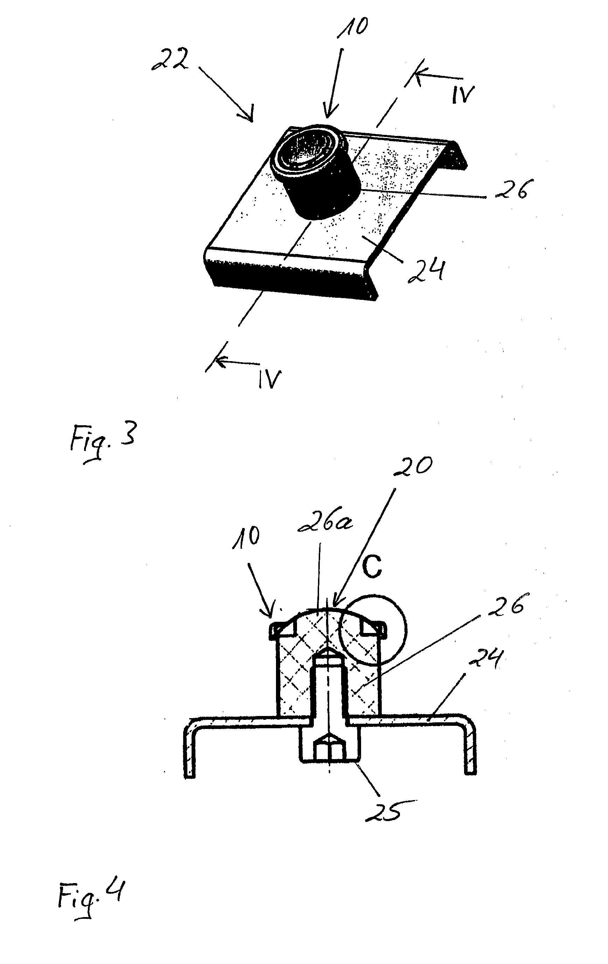

In Figur 3 ist die Haltevorrichtung 10 mit der Amnionmembran 20 an einem Träger 22 angeordnet, der aus einer Grundplatte 24 und einem Tragkörper 26 besteht. Auf den Tragkörper 26 ist die Haltevorrichtung 10 aufgesetzt.

Wie insbesondere aus Figur 4 ersichtlich, ist der Tragkörper 26 an der Grundplatte 24 von deren Unterseite aus mit einer Schraube 25 befestigt, wobei die Grundplatte an zwei gegenüberliegenden Seiten umgebogen ist, so dass die Grundplatte eine Standsicherung für den Tragkörper 26 bildet.

Der Tragkörper 26 ist ein kreiszylindrisch ausgebildeter Körper, der mit einer flachen Stirnseite der Grundplatte 24 flächig aufliegt und an der entgegengesetzten Stirnfläche 26a nach außen gewölbt ausgebildet ist. Die Wölbung des Tragkörpers 26 entspricht dabei einer Kugelkappe mit einem Kugelradius von 12 mm, die sich über dem Kreiszylinder des Tragkörpers 26 mit einem Radius von 8,7 mm wölbt. Der Durchmesser des Kreiszylinders des Tragkörpers 26 ist so gewählt, dass die Haltevorrichtung ab dem Übergang an der Innenfläche 12b von dem formstabilen Innenring 14 auf den weich-elastischen Außenring 12 dem Tragkörper radial nach außen übersteht.

Die Haltevorrichtung 10 ist dabei auf die gewölbte Stirnfläche 26a aufgesetzt, wobei die Amnionmembran 20 mit ihrer Unterseite auf der gewölbten Stirnfläche aufliegt, die in ihrer Kontur weitgehend einem menschlichen Auge entspricht.

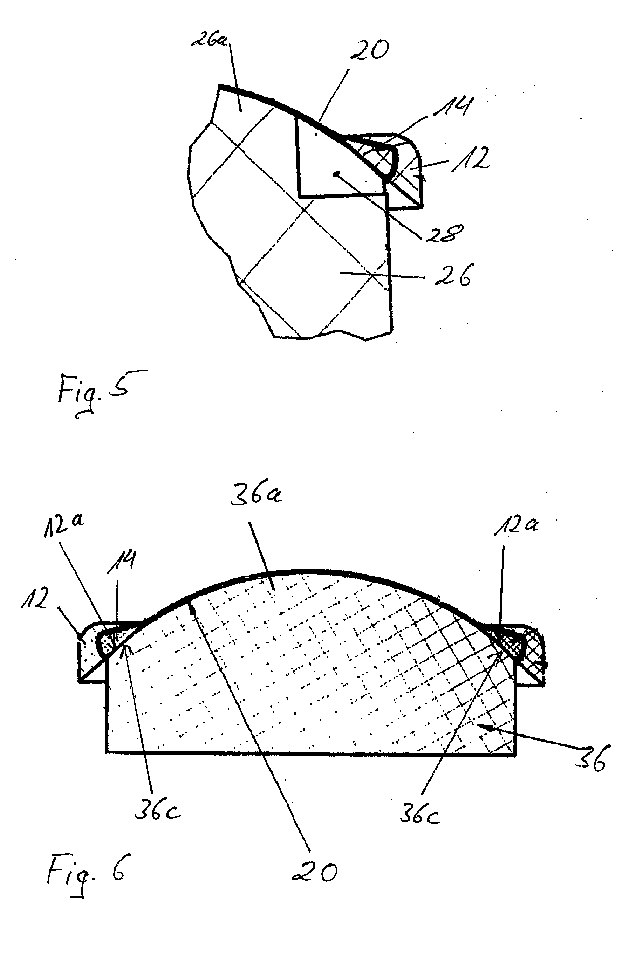

Wie in Figur 5 dargestellt, weist der Tragkörper 26 an der Basis der die Stirnfläche 26a bildenden Kugelkappe eine partiell oder vollständig umlaufende, im Querschnitt weitgehend dreieckförmige Nut 28 auf, die sich im Übergangsbereich zwischen dem zylindrischen Grundkörper und der Kugelkappe des Tragkörpers 26 radial in den Tragkörper 26 erstreckt. Mit dieser Ausbildung liegt die Haltevorrichtung 10 mit den Ringen 12, 14 nur dem Randbereich des Tragkörpers 26 auf, wobei die Amnionmembran 20 der Kugelkappe der Stirnseite 26a aufliegt und auf diese Weise unter einer glättenden Zugspannung steht.

In einer alternativen, in Figur 6 gezeigten Ausführungsform einer Stirnseite 36a eines ansonsten identisch, allerdings ohne eine Grundplatte ausgebildeten Tragkörpers 36 eines Trägers 22 ist die die gewölbte Stirnseite bildende Kugelkappe an Ihrer Basis, also nach außen, in eine an der Innenfläche 12b der an dieser Stelle zur Auflage bringbaren Ringe 12, 14 angepasste Kegelstumpfform 36c überführt, so dass die Ringe 12, 14 mit der gemeinsamen Seiteninnenfläche 12b dem Abschnitt 36c der gewölbten Stirnseite 36a flach zur Anlage gebracht werden können.

Die Amnionmembran 20 kann in der Anordnung in der Haltevorrichtung 10, in der sie zur medizinischen Anwendung in das Auge eingebracht wird, bereits vor dieser Anwendung in einer Nährlösung, auch tiefgefroren, aufbewahrt und/oder für eine biochemische oder gewebeverändernde Manipulation, z. B. für eine Vernetzung mit Proteinen oder für ein Aufbringen von Wachstumsfaktoren, zugänglich gemacht werden.

Bei einer Anordnung der Haltevorrichtung 10 an dem Träger 22 ist die Amnionmembran 10 bei diesen Vorgängen vor der eigentlichen Operation auch definiert positioniert und gegen Beschädigungen geschützt. Die gewölbte Stirnseite 26a, 36a des Tragkörpers 26, 36 schützt die Amnionmembran durch die aufgebrachte Zugspannung vor einer Faltenbildung, wodurch der für die wundheilende Wirkung der Membran notwendige direkte, möglichst faltenfreie Kontakt mit der Hornhaut verbessert ist.

In der Haltevorrichtung 10 und gegebenenfalls in Kombination mit dem Träger 22 wird die Amnionmembran nach dem Aufbewahren und Aufbereiten gebrauchsfertig dem Operateur zur Verfügung gestellt, der die Haltevorrichtung ohne weitere Entnahme- oder Befestigungsschritte in das Auge einbringen kann.

Dadurch dass die Amnionmembran 20 bereits in der Haltevorrichtung 10 aufbewahrt wird, sind die Veränderungen an der Membran deutlich reduziert und die Amnionmembranen weisen eine hohe optische Qualität auf.

Die medizinische Anwendung der erfinderischen Lehre ist bevorzugt im humanen Bereich zu sehen, aber nicht auf diesen beschränkt.

## Patentansprüche

1. Haltevorrichtung (10) für eine Membran (20) für eine medizinische Anwendung am Auge, mit zwei konzentrisch aneinander angeordneten Ringen (12, 14), wobei ein erster Ring als Innenring (14) von einer Aufnahme (12a) an der Innenseite eines zweiten Rings als Außenring (12) aufgenommen ist, wobei die Membran (20) im Bereich dieser Aufnahme (12a) zwischen den zwei Ringen umfänglich aufspannbar und die gemeinsame Öffnung (18) der zwei Ringe (12, 14) überdeckend anordenbar ist, wobei die zwei Ringe eine gemeinsame kegelstumpfförmige Innenfläche (12b) aufweisen und der Innenring (14) weitgehend formstabil und der Außenring (12) elastisch ist, wobei der Außenring (12) den Innenring (14) unter Spannung umschließt und die Membran (20) durch diese Spannung zwischen dem Außenring und dem Innenring halterbar ist,
**dadurch gekennzeichnet,**
**dass** der Außenring (12) eine weitgehend als Dreieck ausgebildete Grundfläche mit einer die Aufnahme (12a) bildenden, keilförmigen Ausnehmung aufweist, mit einer Seite des Dreiecks, die ausgehend von einer seiner Spitzen, teilweise von einer Seite der keilförmigen Ausnehmung gebildet ist und mit einer eine abgerundete Kante (12c) bildenden Spitze im Gegenwinkel dieser Seite.

2. Haltevorrichtung nach Anspruch 1,
**dadurch gekennzeichnet,**
**dass** der Außenring (12) den Innenring (14) an dessen Außen- und Oberseite überdeckend angeordnet ist.

3. Haltevorrichtung nach einem der Ansprüche 1 oder 2,
**dadurch gekennzeichnet,**
**dass** der Außenring (12) an seinem unteren Abschnitt dem Innenring (14) überstehend und mit einer spitz auslaufenden Kante (12d) ausgebildet ist.

4. Haltevorrichtung nach einem der Ansprüche 1 bis 3,
**dadurch gekennzeichnet,**
**dass** der Innenring (14) eine keilförmige Querschnittsfläche aufweist.

5. Haltevorrichtung nach einem der Ansprüche 1 bis 4,
**dadurch gekennzeichnet,**
**dass** die gemeinsame, kegelstumpfförmige Innenfläche (12b) der beiden zusammenwirkenden Ringe (12, 14) einen Öffnungswinkel α im Bereich der Wölbung eines menschlichen Augapfels aufweist.

6. Haltevorrichtung nach Anspruch 5,
**dadurch gekennzeichnet,**
**dass** der Öffnungswinkel α ca. 97° beträgt

7. Haltevorrichtung nach einem der Ansprüche 1 bis 6,
**dadurch gekennzeichnet,**
**dass** der Innenring (14) aus einem Thermoplast besteht.

8. Haltevorrichtung nach einem der Ansprüche 1 bis 7,
**dadurch gekennzeichnet,**
**dass** der Außenring (12) aus Silikon besteht.

9. Träger (22) für eine nach den Ansprüchen 1 bis 8 ausgebildete Haltevorrichtung (10), mit einem Tragkörper (26, 36), wobei der Tragkörper ein Zylinder mit kreisförmiger Querschnittsfläche und einer nach außen gewölbten Stirnseite (26a, 36a) ist, die Wölbung der Stirnseite in etwa der Wölbung eines menschlichen Augapfels im Bereich der Hornhaut entspricht und der Durchmesser der Stirnseite an ihrer Basis mit dem Außendurchmesser des Innenrings (14) der Haltevorrichtung (10) weitgehend identisch ist.

10. Träger nach Anspruch 9,
**dadurch gekennzeichnet,**
**dass** die umfänglichen Randbereiche (36c) der Stirnseite (36a) an deren Basis kegelstumpfförmig ausgebildet sind.

11. Träger nach Anspruch 9,
**gekennzeichnet durch**,
eine umlaufende Nut (28) im Bereich der Basis der Stirnseite (26a), wobei die Nut (28) mindestens die radiale Tiefe der radialen Erstreckung des Innenrings (14) der Haltevorrichtung (10) aufweist.

## Claims

1. Holding device (10) for a membrane (20) for a medical application to the eye, comprising two rings (12, 14) being disposed concentrically with respect to each other, wherein a first ring, which is embodied as an inner ring (14) of a receptacle (12a), is received on the inside of a second ring which is configured as an outer ring (12), wherein said membrane (20) can be disposed in the region of said receptacle (12a) such that it can be clamped circumferentially between the two rings and such that the common opening (18) of the two rings (12, 14) is covered, wherein the two rings feature a common
frusto-conical inner surface (12b), the inner ring (14) is largely dimensionally stable and the outer ring (12) is elastic, wherein the outer ring (12) encloses the inner ring (14) under tension and the membrane (20) can be held between the outer ring and the inner ring by means of said tension,
**characterized in that**
the outer ring (12) features an essentially triangular base with a wedge-shaped recess forming the receptacle (12a), wherein one side of the triangle, starting from one of the vertices thereof, is partially formed by one side of the wedge-shaped recess, and wherein one vertex forms a rounded edge (12c) in opposite angle to said side.

2. Holding device according to claim 1,
**characterized in that**
the outer ring (12) is arranged so as to cover the inner ring (14) on its outside and upper side.

3. Holding device according to any of claims 1 or 2,
**characterized in that**
the outer ring (12) along its lower portion protrudes beyond the inner ring (14) and is embodied with a tapering edge (12d).

4. Holding device according to any of claims 1 to 3,
**characterized in that**
the inner ring (14) features a wedge-shaped cross-sectional surface.

5. Holding device according to any of claims 1 to 4,
**characterized in that**
the common, frusto-conical inner surface (12b) of the two interacting rings (12, 14) features an opening angle α in the region of the curvature of a human eyeball.

6. Holding device according to claim 5,
**characterized in that**
the opening angle is approx. 97°.

7. Holding device according to any of claims 1 to 6,
**characterized in that**
the inner ring (14) is made of a thermoplastic material.

8. Holding device according to any of claims 1 to 7,
**characterized in that**
the outer ring (12) is made of silicone.

9. Support (22) for a holding device (10) being embodied according to any of claims 1 to 8, comprising a support member (26, 36), wherein said support member is a cylinder having a circular cross-sectional surface and a frontal side (26a, 36a) being curved towards the outside, the curvature of the frontal side essentially corresponding to the curvature of a human eyeball in the region of the cornea, and the diameter at the base of the frontal side being largely identical with the outer diameter of the inner ring (14) of the holding device (10).

10. Support according to claim 9,
**characterized in that**
the base of the circumferential edge regions (36c) of the frontal side (36a) features a frusto-conical shape.

11. Support according to claim 9,
**characterized by**
a circumferential groove (28) in the region of the base of the frontal side (26a), wherein the groove (28) has at least the radial depth of the radial extension of the inner ring (14) of the holding device (10).

## Revendications

1. Dispositif de retenue (10) pour une membrane (20) destiné à une application médicale sur l'oeil, comportant deux bagues (12, 14) étant agencées de manière concentrique l'une par rapport à l'autre, dans lequel une première bague étant conçue en tant que bague intérieure (14) d'un logement (12a) est logée à l'intérieur de la seconde bague étant conçue en tant que bague extérieure (12), la membrane (20) pouvant être agencée dans la région dudit logement (12a) de telle manière que celle-ci puisse être serrée circonferentiellement entre les deux bagues et de telle manière que l'ouverture commune (18) des deux bagues (12,14) soit recouverte, les deux bagues présentant une commune surface intérieure (12b) de forme tronconique, la bague intérieure (14) étant sensiblement dimensionnellement stable et la bague extérieure (12) étant élastique, la bague extérieure (12) entourant la bague intérieure (14) sous tension et la membrane (20) pouvant être retenues entre la bague extérieure et la bague intérieure au moyen de ladite tension,
**caractérisé en ce que**
la bague extérieure (12) présente une base essentiellement de forme triangulaire avec un évidement cunéiforme formant le logement (12a), un côté du triangle, partant de l'un de ses sommets, étant formé partiellement par un côté de l'évidement cunéiforme, et un sommet formant un bord arrondi (12c) selon un angle opposé par rapport audit côté.

2. Dispositif de retenue selon la revendication 1,
**caractérisé en ce que**
la bague extérieure (12) est agencée de telle manière que la bague intérieure (14) soit recouverte sur son coté extérieur et sur son coté supérieur.

3. Dispositif de retenue selon la revendication 1 ou 2,
**caractérisé en ce que**
la bague extérieure (12) le long de sa partie inférieure est conçue de façon à dépasser au-delà de la bague intérieure (14) et présente un bord effilé.

4. Dispositif de retenue selon l'une quelconque des revendications 1 à 3,
**caractérisé en ce que**
la bague intérieure (14) présente une surface de section transversale cunéiforme.

5. Dispositif de retenue selon l'une quelconque des revendications 1 à 4,
**caractérisé en ce que**
la surface intérieure (12b) commune de forme tronconique des deux bagues (12, 14) interagissantes présente un angle d'ouverture α dans la région de la courbure d'un globe oculaire humain.

6. Dispositif de retenue selon la revendication 5,
**caractérisé en ce que**
l'angle d'ouverture est d'environ 97°.

7. Dispositif de retenue selon l'une quelconque des revendications 1 à 6,
**caractérisé en ce que**
la bague intérieure (14) est réalisée en matériau thermoplastique.

8. Dispositif de retenue selon l'une quelconque des revendications 1 à 7,
**caractérisé en ce que**
la bague extérieure (12) est faite de silicone.

9. Support (22) pour un dispositif de retenue (10) étant réalisé selon l'une quelconque des revendications 1 à 8, comprenant un élément de support (26, 36), ledit élément de support étant un cylindre qui a une surface de section transversale circulaire et un côté frontal (26a, 36a) étant courbé vers l'extérieur, ladite courbure du côté frontal correspondant sensiblement à la courbure d'un globe oculaire humain dans la région de la cornée, et le diamètre de la base du côté frontal étant largement identique au diamètre extérieur de la bague intérieure (14) du dispositif de retenue (10).

10. Support selon la revendication1,
**caractérisé en ce que**
la base des zones de bord (36c) circonférentielles du côté frontal (36a) est réalisée de forme tronconique.

11. Support selon la revendication 9,
**caractérisé par**
une rainure périphérique (28) dans la zone de la base du côté frontal (26a), ladite rainure (28) ayant au moins la profondeur radiale de l'extension radiale de la bague intérieure (14) du dispositif de retenue (10).
